# EUROPEAN PATENT APPLICATION

(11) **EP 2 261 656 A1**
(43) Date of publication of application: **15.12.2010**
(21) Application number: 09723341.5
(22) Date of filing: 23.03.2009
(51) Int. Cl.: G01N 31/00, G01N 21/78, G01N 31/22, G01N 33/84

(54) **DRY TEST INSTRUMENT, METHOD OF MEASURING METAL AND METHOD OF PRODUCING DRY TEST INSTRUMENT**

(30) Priority: 21.03.2008 JP 2008074745
(71) Applicant: ARKRAY, Inc., Kyoto-shi, Kyoto 601-8045 (JP)
(72) Inventor: OKAMOTO, Kazuhiro, Kyoto-shi Kyoto 601-8045 (JP); FUKUTA, Isao, Kyoto-shi Kyoto 601-8045 (JP)
(74) Representative: Golding, Louise Ann
(86) International application number: PCT/JP2009/055715
(87) International publication number: WO 2009/116669

(57) **Abstract**

The present invention provides a dry testing tool with which a metal in the various types of samples such as biological samples, food, and the like can be measured very easily, rapidly, high sensitively, and specifically by a simple colorimetric method and a method for measuring the metal. The dry testing tool of the present invention is a dry testing tool for measuring a metal in a sample, including a porous support and a chelate dye, wherein the chelate dye is bound to the porous support by a hydrophobic bond. The measuring method of the present invention is a method for measuring a metal in a sample, including the steps of: forming a composite of the chelate dye and the metal in the sample by supplying the sample to the porous support to which a chelate dye is bound by a hydrophobic bond; and detecting the metal in the sample using a developed color of the composite.

## Description

### Technical Field

The present invention relates to a method for measuring a metal, especially a heavy metal, that is present in a trace amount in a sample, and a dry testing tool.

### Background Art

Among metals, light metals such as Na, K, Ca, Mg, Zn, Cu, Fe, and the like are vital metals in order for living things to survive. However, heavy metals such as mercury (Hg), lead (Pb), cadmium (Cd), arsenic (As), and the like are metals having very high toxicity for living things, even in trace amounts.

As the civilization has developed, copper and iron appearing on the surface of the ground were mined and had been used for producing bronze wares and iron wares. On the other hand, since heavy metals are mostly present in the deep earth, the amounts thereof obtained from the surface of the ground by mining were fortunately very small. Therefore, heavy metals had been utilized for only particular applications, and it was not a big problem. However, since the Industrial Revolution, coal, petroleum, iron ore, bauxite, gold, silver, copper, and the like became essential metals for progress in industry, whereby metals came to be mined from the deep earth. Consequently, as byproducts of the metals, heavy metals such as mercury (Hg), lead (Pb), cadmium (Cd), arsenic (As), and the like also have been mined, and used for the various products. For example, Hg has been utilized for mercury thermometers, fluorescent lamps, dental fillings (mercury amalgam), and refining of gold and silver. However, there are cases where pollution-caused diseases were developed due to the fact that mercury was used as a synthetic catalyst for aldehyde acetate, was changed to organic mercury in the course of synthesis, and was released to rivers as sewage. The large amount of lead (Pb) has been used as a solder of an electric circuit, and also as a material for raising octane numbers by mixing it into gasoline, and a developer of paint. Further, cadmium (Cd) has been used as a dry cell or a semiconducting material, and arsenic (As) has been used as an agricultural chemical, an ant killer, and a semiconducting material.

Most of the products in which these metals are used eventually become waste and are burned or deposited in landfills. However, unlike synthetic compounds, heavy metals do not change even though they are burned, and they eventually are dissolved into rain water and are returned to the surface of the ground even though they turn into gas. Landfilled heavy metals also are dissolved gradually into ground water by acid rain and are returned to the surface of the ground as plants or well water containing them. The heavy metals returned to the water of the surface of the ground are absorbed by plants and consumed by fish and selfish and stored therein. Further, the heavy metals eventually are stored in humans who are at the top of the food chain. Ministry of Health, Labour and Welfare provides the emergency information and alerts to women who are to be mothers and pregnant women not to eat deep-sea fish such as tunas, whales, dolphins, alfonsinos, and the like that are at the top of the food chain in fish and selfish.

Heavy metals chronically stored in a human body strongly bind to a SH group (residue of cysteine) of a protein, cause important conformation of the protein to be changed, and cause various kinds of disorders (disorders of metabolism) to be developed over a long period of time.

When the heavy metals are, for example, bound to a membrane protein or a transmitter of neurons, various kinds of neurologic symptoms of disorders such as dementia, depression, autism, Down's syndrome, and the like are developed. Further, when the heavy metals are bound to a protein of a white blood cell in the immune system, abnormal allergies such as atopic dermatitis, food allergy, pollinosis, rhinitis, and the like are developed. Furthermore, when the heavy metals act directly on a protein or a gene in a gene expression system in cells, a normal gene expression does not occur, and genetic diseases or malignant transformation of cells are progressed. These symptoms tend to increase not only especially in advanced countries, but also in advancing countries in the world.

Likewise, besides heavy metals, as a cause of developing these disorders, environmental pollution caused by the various kinds of artificial objects produced with the progress in industry such as, especially, endocrine-disrupting chemicals, agricultural chemicals, additives, general synthetic substances, and the like has been spread. It has been considered that the wide range of symptoms could be developed in anyone because the complicated synergistic action between residual storage of these artificial objects (environmental pollutant) and residual storage of the heavy metals also is generated, and further, there are individual differences of sensitivity against these objects.

As the nature of theses very trace amounts of heavy metals or the various types of environmental pollutants, there are many cases where acute toxicity is not shown, and symptoms gradually progress due to accumulation of the trace amounts of heavy metals over a long period of time. These symptoms are diagnosed as aging or genetic factor, and studies or developments of definite treatments have not been promoted so far. The fact is also that the progress in industry or convenience and the cost reduction or profit increase have been top priority, and countries and their governments, and also people themselves have turned blind eyes to a certain level of hazard. There is a possibility that even though we perceive the risk for discharging the heavy metals, there is the business mind that continuing this study prevents progress in industry because the progress in industry has taken precedence, and it is difficult to prove the definite cause-and-effect relationship because of chronic diseases, and researchers also have given importance to the positive aspect of the heavy metals and avoided taking the negative aspect seriously.

In recent years, with the rapid progress in technology of analyzing the trace amount of content and also the result of steady basic studies by many researchers, it has been proven that the heavy metals have been stored in many living things. For example, in Japan, Minamata disease (Hg contamination), itai-itai disease (Cd contamination), and the like are known as pollution-caused diseases having acute toxicity caused by industry. Further, even today, the mechanism of the influence of the heavy metals on living things and the treatment of the diseases have been studied.

Further, in the United States, the risk of heavy metals stored in biological bodies has been studied, and the detoxification treatment for causing the heavy metals to be excreted selectively from the biological bodies has been provided for 800,000 people every year. Specifically, the oral administrations of DMSA (DMSA is approved by FDA) and DMPA are known as the chelation treatment. National Institutes of Health (NIH) conducts clinic-epidemiological evaluations relating to the utility of the chelation treatment with funding of 30,000,000 dollars. This clinic-epidemiological evaluation is intended to excrete Ca from an artery by a chelation treatment in calcification caused by stored Ca in an artery, which is a cause of heart diseases that are progressing rapidly in the U.S. It is pointed out that the evaluations inherently are intended to ameliorate Alzheimer and dementia. Also in Japan, the detoxification treatments have been conducted in some medical institutions, and quantitative determinations of heavy metals in hair or urine have been conducted. The measurements of heavy metals are conducted by the ICP-MS method (Inductively Coupled Plasma Mass Spectrometer) at the foreign and domestic testing institutions, and the risk of the heavy metals and the importance of early treatment are being acknowledged.

However, the environment for measuring harmful heavy metals is poor in Japan. When people ask for measuring the heavy metals in biological samples such as hair, blood, urine, and the like in Japan, a procedure as below is required, for example. That is, first, diagnosis is received in an affiliated clinic not covered by insurance. Next, a so called challenge test is conducted in which 500 mg of DMSA, which is an oral detoxifying agent, is administered orally on an empty stomach after excreting the first morning urine, and urine that is excreted six hours after the oral administration is collected. Further, the collected urine is sent to a laboratory in U.S., and a measurement by the ICP-MS method (Inductively Coupled Plasma Mass Spectrometer) is conducted using the collected urine. The challenge test has a big problem that the charge to take the challenge test for one time is several thousands yen, which is expensive, and it takes from weeks to one month to receive the measurement result by the ICP-MS method. Generally, an excretion amount of heavy metals from a living body excreted to urine reaches the maximum amount after three to six hours from conducting the challenge test. Thus, the above-described measuring method is not sufficient to determine the excretion peak or the treatment effect. After a challenge test, when the excretion amount of a heavy metal is large, a treatment strategy for the detoxification treatment is decided. However, in fact, there is a disadvantage that the strategy cannot be decided until receiving the result, i.e., after one month.

Further, in the ICP-MS method that is the current mainstream of measuring a heavy metal in a biological sample or food, the complicated pretreatment where an organic substance is degraded to an inorganic substance by heating the biological sample with nitric acid, sulfuric acid, hydrogen peroxide, or the like is required. After the pretreatment, the metal is brought into the plasma state by microwave and measured with a mass spectrometer, whereby the respective atoms can be measured with high sensitivity. However, the inductively coupled plasma mass spectrometer used in the ICP-MS method is expensive, whereby it is difficult to employ the spectrometer in small-and-medium sized clinical institutions, for example.

Considering the foregoing case, the various kinds of methods for measuring a heavy metal in a biological sample or food by a simple colorimetric method have been devised.

For example, Patent Citation 1 describes a metal ion detection film that is a membrane filter coated with fine particles of a metal ion detecting reagent that has water insolubility or poor water solubility and is soluble in a hydrophobic solvent. However, at the time of measurement, 100 ml of the solution is required to be suction-filtered or pressure-filtered, whereby a big pump is required to be used.

The method described in Patent Citation 2 is a method for detecting an absorbance of arsenic with high sensitivity. The method is a method in which arsenomolybdic is formed by causing arsenic to react with a molybdic acid, the formed arsenomolybdic is caused to react with a triarylmethane dye (ethyl violet), and color is caused to be developed at 530 nm. According to this method, it is possible to conduct the detection with the lowest detection sensitivity of up to 1 µg of arsenic/kg (or µg/L).

The methods described in Patent Citations 3 and 4 are methods for measuring an oxidation current value of heavy metal ions by causing a heavy metal in a sample to be trapped on the chelate resin membrane, concentrating it by various types of methods, and thereafter, causing it to be eluted by the solvent and causing an electrode to be in contact with them.

The method described in Patent Citation 5 is a trace metal measuring method for measuring a change in absorbance caused by a complex forming reaction of porphyrin-nucleus introduced polymers and metal ions, the porphyrin-nucleus introduced polymers being obtained by conducting radical copolymerization of a porphyrin compound having a vinyl group with a radical polymerizable monomer. In Examples of Patent Citation 5, the measuring method using a porphyrin-nucleus introduced gel is disclosed.

The kit described in Patent Citation 6 is a test kit for conducting a colorimetric method of a metal ion concentration in an aqueous solution, and also a reagent kit containing the sufficient amount of water-soluble base for solubilizing a dithizone reagent and dithizone in a test aqueous solution and an iron-soluble compound. Not only the method for measuring a solution system, but also, in the one embodiment, the distributed reagent in a layer on a multilayer analyzing element such as a dry-reagent test slide is disclosed.

The Patent Citation 7 relates to an enzyme amplified competitive assays for metal ions and a sandwich chelation assay for the same. Specifically, a chelator chelating a metal is immobilized on a solid support through a protein, and the chelator can be used for a competitive assay that relies on a competitive inhibition by an objective metal ion in a sample.

Patent Citation 1: JP 2005-274146 A
Patent Citation 2: JP 2007-24634 A
Patent Citation 3: JP 2006-112789 A
Patent Citation 4: JP 2006-189400 A
Patent Citation 5: JP No. 3898720
Patent Citation 6: JP H05(1993)-232028 A
Patent Citation 7: JP H09(1997)-505667 A

### Disclosure of Invention

Since metals, especially, heavy metals are present only in very trace amounts in a sample, a highly sensitive measurement is required to be conducted. Further, to conduct a simple measurement, it is required that metals, especially heavy metals, are caused to be released with mild conditions without conducting wet ashing by heating the sample with a strong acid. Furthermore, since many metal species are generally present in a sample, especially in a biological sample, it is required that the specificity for measuring only the target metal species is increased.

Hence, the present invention is intended to provide a dry testing tool by which a metal in the various types of samples such as biological samples, food, and the like can be measured very easily, rapidly, high sensitively, and specifically by a simple colorimetric method and a method for measuring the metal.

In order to solve the aforementioned problems, the dry testing tool of the present invention is a dry testing tool for measuring a metal in a sample, including a porous support and a chelate dye, wherein the chelate dye is bound to the porous support by a hydrophobic bond.

The measuring method of the present invention is a method for measuring a metal in a sample, including the steps of: forming a composite of a chelate dye and a metal in a sample by supplying the sample to the porous support to which a chelate dye is bound by a hydrophobic bond; and detecting the metal in the sample using developed color of the composite.

The producing method of the present invention is a method for producing the dry testing tool of the present invention, including the steps of: causing a chelate dye to be bound to a porous support by a hydrophobic bond by causing a solution in which the chelate dye is dissolved to be in contact with a porous support having a hydrophobic region; and drying the porous support.

In order to solve the aforementioned problems, the inventors of the present invention conducted earnest studies to provide high sensitivity and specificity in a simple colorimetric method that can be conducted easily and rapidly. As a result of the earnest studies, the inventors of the present invention found out the fact that a chelate dye having high specificity is fixed to the porous support by a hydrophobic bond, a heavy metal is concentrated by specifically trapping with ligands of the chelate dye, and color is developed. Thus, the two tasks of providing high sensitivity and specificity could be solved at the same time, and thereby the present invention was completed.

According to the dry testing tool or the method for measuring a metal in a sample of the present invention, a target metal can be measured specifically very easily, rapidly, and with high sensitivity by the simple colorimetric method. The present invention can be, for example, applied to samples such as a biological sample, food, and the like. However, the present invention is not limited thereto and can be applied to the various types of samples.

### Brief Description of Drawings

[FIG. 1] FIGs. 1A to 1D are schematic views showing an example of a dry testing tool of the present invention. FIG. 1A is a plane view, FIG. 1B is a cross-section view, FIG. 1C is a bottom plane view, and FIG. 1D is a perspective view.
[FIG. 2] FIGs. 2A to 2D are schematic views showing another example of the dry testing tool of the present invention. FIG. 2A is a plane view, FIG. 2B is a cross-section view, FIG. 2C is a bottom plane view, and FIG. 2D is a perspective view.
[FIG. 3] FIG. 3A is a perspective view showing yet another example of the dry testing tool of the present invention. FIG. 3B is a conceptual cross sectional view at the time of using the dry testing tool.

### Best Mode for Carrying out the Invention

### [Dry testing tool]

The dry testing tool of the present invention is, as described above, a dry testing tool for measuring a metal in a sample, including a porous support and a chelate dye, wherein the chelate dye is bound to the porous support by a hydrophobic bond. The dry testing tool of the present invention is, for example, preferably a dry testing tool, wherein, by supplying the sample to a surface of the porous support, the metal in the sample is bound to the chelate dye by a chelate bond and a composite of the chelate dye and the metal is formed on the surface of the porous support.

The porous support is not particularly limited, and is, for example, preferably, at least one selected from the group consisting of filter papers, sheets, membranes, nonwoven fabrics, woven fabrics, fabrics, and sintered bodies.

Further, a material of the porous support is, for example, preferably at least one selected from the group consisting of cellulose, a cellulose derivative, glasses, and a polymer. The cellulose derivative is not particularly limited, and is, for example, preferably at least one selected from the group consisting of cellulose nitrate, cellulose acetate, and mixed cellulose. Furthermore, the polymer is not particularly limited, and is, for example, preferably at least one selected from the group consisting of polycarbonates, polypropylenes, polytetrafluoroethylenes, polyethersulfones, polystyrenes, and polyesters.

The dry testing tool of the present invention is, for example, preferably a dry testing tool further including a porous filter, wherein the porous filter is formed on an upper surface of the porous support, a noise content in the sample is captured by the porous filter by supplying the sample on the upper surface of the porous filter, and the sample passing through the porous filter is supplied to the surface of the porous support. According to the dry testing tool of the present invention, it is possible to conduct the measurement that is insusceptible to the noise content, and further, by previously removing the noise content by capturing it with the porous filter, the influence of the noise content can be further reduced. It is to be noted that, in this case, the developed color of the porous support is, for example, preferably observed from the opposite side (the lower surface side) of the porous filter. It is for the reason that there is a case where the developed color of the porous support cannot be observed from the porous filter side (the upper surface side) because the porous filter is opaque or the like. The material of the porous filter is not particularly limited, and is, for example, the same as that of the porous support. The porous filter may or may not contain the chelate dye. However, in order to achieve the aforementioned object, the porous filter preferably contains the different type of chelate dye from that of the porous support.

The chelate dye is not particularly limited, and is, preferably a chelate dye that specifically forms a chelate composite with an objective metal from the viewpoint of the measurement sensitivity or the like. For example, when the objective metal is mercury, the chelate dye is not particularly limited, and is, more preferably at least one selected from the group consisting of dithizone compounds, thiomichler's compounds, thiothenoyltrifluoroacetone, diphenylcarbazone, zincon, derivates of PADAP, and N-benzoyl-N-phenyl-hydroxylamine, and yet more pferabaly at least one selected from the group consisting of dithizone, thiomichler's ketone, thiothenoyltrifluoroacetone, diphenylcarbazone, zincon, 2-(5-bromo-2-pyridylazo)-5-diethylaminophenol (5-Br-PADAP), and N-benzoyl-N-phenyl-hydroxylamine. When the objective metal is cadmium, the chelate dye is particularly preferably at least one selected from the group consisting of 2-(5-bromo-2-pyridylazo)-5-diethylaminophenol (5-Br-PADAP), 1-(2-pyridylazo)-2-naphthanol (PAN), dithizone, eriochrome black T, and chromazurol S. When the objective metal is aluminum, the chelate dye is particularly preferably at least one selected from the group consisting of stilbene-4,4'-bis(1-azo)-3,4-dihydroxybenzene-2,2'-disulfonic acid (stilbazo), chromazurol S, N-phenylbenzohydroxamic acid (BPA), aurine tricarboxylic acid ammonium salt (another name: aluminon), and phenylfluorone. When the objective metal is zinc, the chelate dye is particularly preferably at least one selected from the group consisting of 2-(5-bromo-2-pyridylazo)-5-diethylaminophenol (5-Br-PADAP), 2-(5-bromo-2-pyridylazo)-5-(N-propyl-N-sulfopropylamino)phenol (5-Br-PAPS), dithizone, 2-(5-nitro-2-pyridylazo)-5-(N-propyl-N-sulfopropylamino)phenol (Nitro-PAPS), PAN, 4-(2-pyridylazo)resorcinol (PAR), phenylfluorone, and zincon. When the objective metal is lead, the chelate dye is particularly preferably at least one selected from the group consisting of 5,10,15,20-tetraphenyl-21H,23H-porphinetetrasulfonic acid, disulfuric acid (TPPS), dithizone, bromopyrogallol red (BPR), PAR, and xylenol orange. When the objective metal is copper, the chelate dye is particularly preferably at least one selected from the group consisting of PAN, PAR, PR, TMPyP, XO, bathocuproine, and dithizone. When the objective metal is cobalt, the chelate dye is particularly preferably 2-nitroso-1-naphthol. When the objective metal is chromium, the chelate dye is particularly preferably diphenylcarbazide.

Preferably, a surface of the porous support includes at least a hydrophobic region, and the chelate dye is bound thereto by a hydrophobic bond. For example, the hydrophobic region preferably has a hydrophobic group. The hydrophobic group is not particularly limited, and is, for example, more preferably at least one selected from the group consisting of alkyl groups, nitro groups, acyl groups, cycloalkyl groups, unsaturated hydrocarbon groups, and phenyl groups. More preferably, the hydrophobic region is formed by, for example, introducing the hydrophobic group to the porous support or coating the porous support with a hydrophobic substance.

The dry testing tool of the present invention is, for example, preferably a dry testing tool further including a water absorbing layer, wherein the porous support and the water absorbing layer are at least partially in contact with each other, and the sample passing through the porous support can be drawn into the water absorbing layer. The water absorbing layer is not particularly limited, and is, for example, more preferably, at least one selected from the group consisting of filter papers, glass fiber filter papers, nonwoven fabrics, sponges, and lattice structures showing a capillary action, or the combination thereof.

The dry testing tool of the present invention having the water absorbing layer is, for example, more preferably a dry testing tool, wherein when a surface of the porous support to which a chelate dye is bound is an upper surface, the water absorbing layer is placed on the lower surface of the porous support, and the porous support and the water absorbing layer are at least partially in contact with each other. Further, this dry testing tool is, for example, yet more preferably a dry testing tool further including a holder provided with a through bore, wherein the porous support and the water absorbing layer are fixed with the holder, the through bore of the holder allows the sample to be passed through from the upper side thereof, and a developed color intensity of a composite of the chelate dye and the metal formed on a surface of the porous support can be measured.

The dry testing tool of the present invention having the water absorbing layer is, for example, more preferably, a dry testing tool, wherein the water absorbing layer is a ring-shaped water absorbing layer provided with a through bore, and when a surface of the porous support to which a chelate dye is bound is a lower surface, the water absorbing layer is placed on the lower surface of the porous support, and the porous support is in contact with the water absorbing layer as covering the through bore part of the ring-shaped water absorbing layer. This dry testing tool is, for example, yet more preferably a dry testing tool further including a holder, wherein the porous support and the water absorbing layer are fixed with the holder, the holder allows the sample to be passed through from the upper side thereof to the porous support, and a developed color intensity of a composite of the chelate dye and the metal formed on the surface of the porous support can be measured from a lower side thereof through the through bore part of the water absorbing layer.

### [Measuring method]

The measuring method of the present invention is, as described above, a method for measuring a metal in a sample, including the steps of: forming a composite of a chelate dye and a metal in a sample by supplying the sample to the porous support to which a chelate dye is bound by a hydrophobic bond; and detecting the metal in the sample using the developed color of the composite.

The measuring method of the present invention is, for example, preferably a measuring method, where in the detection step, a developed color intensity of the composite is measured. In the detection step, more preferably, a concentration or an amount of the metal in the sample is measured based on the developed color intensity of the composite, for example. The method for measuring a concentration or an amount of the metal is not particularly limited, and is, for example, more preferably, a measuring method, wherein the developed color of the composite is detected by a visual check, and the concentration of the metal is measured by comparing the developed color with a standard colorimetric table. Further, the method for measuring a concentration or an amount of the metal is, for example, yet more preferably a measuring method in which a reflectance of the porous support is measured, and the concentration of the metal is calculated from a calibration curve.

Tools, systems, and the like used for conducting the measuring method of the present invention are not particularly limited. However, using the dry testing tool of the present invention is preferred. The measuring method of the present invention is, for example, preferably a measuring method, wherein the dry testing tool of the present invention is used, and the sample is supplied to the porous support in the dry testing tool.

Further, the measuring method of the present invention is, for example, preferably a measuring method, wherein in the forming step, the porous support is immersed in the sample, and then is taken out after the elapse of a certain period of time. In this case, for example, more preferably, the porous support is fixed on a tip of a reed-shaped supporting substrate, and the porous support fixed on the supporting substrate is immersed in the sample.

### [Producing method]

The method of producing the dry testing tool of the present invention is not particularly limited. However, the dry testing tool of the present invention is preferably produced by the producing method of the present invention. As described above, the producing method of the present invention is a method for producing the dry testing tool of the present invention, including the steps of: causing a chelate dye to be bound to a porous support by a hydrophobic bond by causing a solution in which the chelate dye is dissolved to be in contact with a porous support having a hydrophobic region; and drying the porous support. A method for causing the solution to be in contact with the porous support is not particularly limited, and is, for example, preferably, at least one method selected from the group consisting of impregnation methods, coating methods, printing methods, spraying methods, and ink-jet methods. The solvent is not particularly limited, and is, for example, preferably, an organic solvent, or a mixed solvent of an organic solvent and water.

### [Embodiment]

Hereinafter, an embodiment of the present invention will be further specifically explained.

In the present invention, the sample that is to be an objective sample is not particularly limited, and is, for example, a fluid sample. The fluid sample is not particularly limited, and examples thereof include a liquid sample, a sol sample, and the like. Further, the sample can be, for example, a gel sample or the like. Hereinafter, the present invention will be described with reference to an example mainly using the liquid sample as the sample. However, the sample may be the sol sample instead of the liquid sample.

In the dry testing tool of the present invention, the porous support is not particularly limited as described above. The porous support may be, for example, a filter paper, a glass fiber filter paper, a nonwoven fabric, a membrane (cellulose nitrate, cellulose acetate, mixed cellulose, polycarbonate, polypropylene, polyester, or mixed membrane thereof), a fabric, a woven fabric, a sintered object, or a porous sheet mixed or laminated thereof. The mixed cellulose is not particularly limited, and can be, for example, a mixture of cellulose acetate and cellulose nitrate or the like.

An average pore size of the porous support is not particularly limited, and is preferably in a range of from 0.2 to 10 µm, more preferably in a range of from 0.2 to 3 µm, and particularly preferably 3 µm. The average pore size is preferably not too large from the viewpoint of capturing an objective metal effectively in the chelate dye. The average pore size is preferably not too small from the viewpoint of a passing speed of a liquid sample to the porous support, prevention of clogging, and the like. It is to be noted that, for example, in the case of the porous support whose average pore size cannot be defined, the pore size thereof is not particularly limited. However, the porous support preferably has the pore size according to the above-described pore size.

Further, in the dry testing tool of the present invention, from the viewpoint of the measurement sensitivity, it is preferable that, for example, a chelate dye capable of forming a chelate composite that is specific to each type of metal contained in a liquid sample is selected. For example, when a metal contained in a liquid sample is mercury, the chelate dye is preferably selected from dithizone compounds or thiomichler's compounds (for example: thiomichler's ketone). In addition, as the chelate dye, a hydrophobic chelate dye can be selected as appropriate from the chelate dyes used as a color-developing reagent forming a chelate complex with heavy metal ions and can be used, for example. Examples of such a chelate dye include: dithizone, thiomichler's ketone, thiothenoyltrifluoroacetone, diphenylcarbazone, zincon, 2-(5-bromo-2-pyridylazo)-5-diethylaminophenol (5-Br-PADAP), and N-benzoyl-N-phenyl-hydroxylamine as a chelate dye forming a chelate complex with mercury; 2-(5-bromo-2-pyridylazo)-5-diethylaminophenol (5-Br-PADAP), 1-(2-pyridylazo)-2-naphthanol (PAN), dithizone, eriochrome black T, and chromazurol S as a chelate dye forming a chelate complex with cadmium; stilbene-4,4'-bis(1-azo)-3,4-dihydroxybenzene-2,2'-disulfonic acid (stilbazo), chromazurol S, N-phenylbenzohydroxaxamic acid (BPA), aurine tricarboxylic acid ammonium salt (another name: aluminon), and phenylfluorone as a chelate dye forming a chealte complex with aluminum; 2-(5-bromo-2-pyridylazo)-5-diethylaminophenol (5-Br-PADAP), 2-(5-bromo-2-pyridylazo)-5-(N-propyl-N-sulfopropylamino)phenol (5-Br-PAPS), dithizone, 2-(5-nitro-2-pyridylazo)-5-(N-propyl-N-sulfopropylamino)phenol (nitro-PAPS), PAN, 4-(2-pyridylazo)resorcinol (PAR), phenylfluorone, and zincon as a chelate dye forming a chealte complex with zinc; 5,10,15,20-tetraphenyl-21H,23H-porphinetetrasulfonic acid, disulfuric acid (TPPS), dithizone, bromopyrogallol red (BPR), PAR, and xylenol orange as a chelate dye forming a chelate complex with lead; PAN, PAR, PR, TMPyP, XO, bathocuproine, and dithizone as a chelate dye forming a chelate complex with copper; 2-nitroso-1-naphthol as a chelate dye forming a red complex with cobalt (II); diphenylcarbazide as a chelate dye forming a purple-red complex with chrome (VI); thiothenoyltrifluoroacetone as a chelate dye forming a yellow complex with both mercury (I) and mercury (II), and the like. In the present invention, "dithizone compound" is dithizone or a derivate thereof. The derivate of dithizone is, for example, a compound in which at least one hydrogen atom of dithizone is substituted with a substituent. Dithizone or a derivate thereof is, for example, preferably a compound represented by the following chemical formula (1). In the following chemical formula (1), each of R¹ and R² is a hydrogen atom or any substituent and may be one or plural and identical to or different from each other. Examples of R¹ and R² include hydrocarbon groups such as a straight-chain or branched-chain alkyl group, a straight-chain or branched-chain alkenyl group, a straight-chain or branched-chain alkylene group, and the like. The carbon number of the hydrocarbon groups is not particulally limited, and is, for example, from 1 to 20.

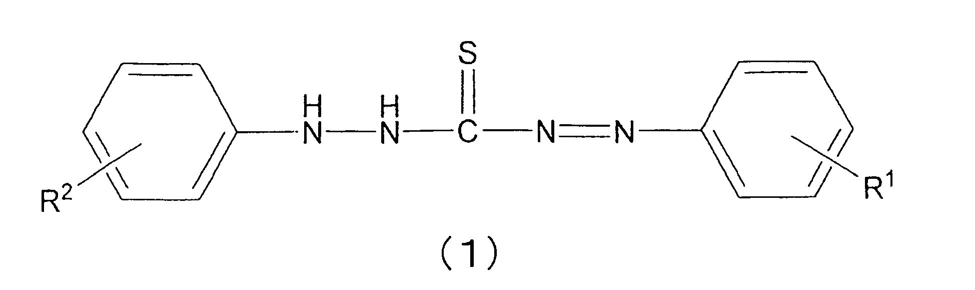

In the dry testing tool of the present invention, the surface to which a hydrophobic bond is formed with the chelate dye is required to have hydrophobicity in order to form a hydrophobic bond between the porous support and a chelate dye. A method for causing the surface of the porous support to have hydrophobicity is not particularly limited. The method may be, for example, a method in which the surface of the porous support has hydrophobicity by a hydrophobic region derived from composition itself of the porous support. Further, the method may be a method in which hydrophobicity of the surface of the porous support is obtained by introducing a hydrophobic group to a hydrophilic region based on the composition of the porous support or further introducing a hydrophobic group to a hydrophobic region of the porous support. Furthermore, the method may be a method in which hydrophobicity of the surface of the porous support is obtained by coating the surface of the porous support with a hydrophobic substance. These methods can be used alone or in a combination of two or more of them. When a hydrophobic group is introduced to the porous support, the porous support and the hydrophobic group may be bound to each other by a covalent bond or a noncovalent bond. When the surface of the porous support is coated with a hydrophobic substance, the surface of the porous support and the hydrophobic substance may be bound to each other by a covalent bond or a noncovalent bond. Introducing a hydrophobic group to the porous support means introducing the hydrophobic group to the interior of the porous support, for example. Coating the surface of the porous support with a hydrophobic substance means coating only the surface of the porous support with the hydrophobic substance, for example. However, the distinction between them is not always sharp. Introducing a hydrophobic group or coating with a hydrophobic substance leads a chelate dye easily to be bound to the surface of the porous support by a hydrophobic bond, and it is preferred from the viewpoint of providing higher measurement sensitivity and specificity to a dry testing tool.

The hydrophobic group is not particularly limited and can be, for example, any of an alkyl group, a nitro group, an acyl group, a cycloalkyl group, an unsaturated hydrocarbon group, and a phenyl group, or the combination thereof. The alkyl group is not particularly limited, and is, for example a straight-chain or branched-chain alkyl group with a carbon number of from 1 to 20. Specific examples thereof include a methyl group, an ethyl group, a n-propyl group, an isopropyl group, a n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, and the like. The acyl group is not particularly limited, and is, for example, a saturated or unsaturated and straight-chain or branched-chain acyl group with a carbon number of from 1 to 20. Specific examples thereof include a formyl group, an acetyl group, a propionyl group, a butyryl group, a hexanoyl group, an octanoyl group, a lauroyl group, a palmitoyl group, a stearoyl group, an oleoyl group, and the like. It is to be noted that an acyl group with a carbon number of 1 is referred to as a formyl group. The cycloalkyl group is not particularly limited, and is, for example, a cycloalkyl group with a carbon number of from 1 to 20. The unsaturated hydrocarbon group is not particularly limited, and is, for example, an unsaturated hydrocarbon group with a carbon number of from 1 to 20. Examples thereof include an alkenyl group, an alkynyl group, an aryl group, and the like. These hydrophobic groups may be, for example, substituted with the other substituent having hydrophobicity such as an alkyl group and the like.

The hydrophobic substance is not particularly limited, and is, for example, a substance having a structure in which a hydrophilic group such as an amino group, a hydroxyl group, a carboxyl group, or the like is bound to the hydrophobic group. Specific examples thereof include octadecylamine, octylamine, the various types of surfactants, and the like.

The method in which hydrophobicity of the surface of the porous support is obtained by introducing a hydrophobic group to a hydrophilic region besed on the composition of the porous support and the method in which hydrophobicity of the surface of the porous support is obtained by coating the surface of the porous support with a hydrophobic substance are not particularly limited. For example, there is a method in which octadecylamine is fixed by immersing a porous support of mixed cellulose formed from cellulose acetate and cellulose nitrate in an aqueous solution of octadecylamine and drying the porous support. Specific examples of the mixed cellulose include Mixed Cellulose Esters (MCE) Membrane Filters (product name) produced by Advantech Co., Ltd. and the like. The aqueous solution of octadecylamine is, for example, an aqueous solution of a salt such as octadecylamine hydrochloride or the like. The immersing time of the porous support in the octadecylamine aqueous solution also is not particularly limited, and can be set as appropriate. The drying temperature and the drying time of the porous support are also not particularly limited, and can be set as appropriate. For example, the porous support may be air-dried at room temperature.

The reason why the surface of the porous support has hydrophobicity by coating (or fixing) the mixed cellulose porous support with octadecylamine is not always clear. For example, it is considered that the reason is that an amino group in octadecylamine is noncovalently bound to a nitro group that is on the surface of the mixed cellulose porous support, and the hydrophobic octadecy group is oriented toward the surface of the mixed cellulose porous support. It is presumed that the nitro group is noncovalently bound to the amino group because there are cases where the nitro group acts as a hydrophobic group and as a hydrophilic group. However, these are merely one example of a presumable mechanism, and do not at all limit the present invention.

The method for producing the dry testing tool of the present invention is not particularly limited as described above. The formation of a hydrophobic bond between the porous support and the chelate dye can be achieved by, for example, just causing a hydrophobic bond to form by applying the chelate dye solution to the porous support, and thereafter, drying the porous support. A method for applying the chelate dye to the porous support is not particularly limited and can be, for example, any of an impregnation method, a coating method, a printing method, a spraying method, and an ink-jet method or the combination thereof. The solvent of the chelate dye solution also is not particularly limited and can be, for example, an organic solvent or a mixed solvent of an organic solvent and purified water. The organic solvent is not particularly limited, and examples thereof include: alcohols such as methanol, ethanol, propanol, isopropanol, butanol, isobutanol, and the like; ketones such as acetone, methyl isobutyl ketone, and the like; esters such as ethyl acetate and the like; and the like. The concentration of the chelate dye in the solution is not particularly limited and can be set as appropriate. When the porous support is immersed in the solution, the immersing time is also not particularly limited and can be set as appropriate. The temperature and the time for drying the porous support also are not particularly limited. For example, the porous support may be air-dried at room temperature.

In the dry testing tool of the present invention, for example, an additive except for the chelate dye may be fixed to the surface of the porous support or may be introduced to the inside of the porous support. The additive is not particularly limited, and examples thereof include a binder (fixing agent), a surfactant, urea, and the like. The binder functions further to strongly fix the chelate dye to the porous support and cause the dry testing tool further to increase the sensitivity and the specificity, for example. These additives can be used alone or in a compination of plural types of them. A method for fixing these additives to the surface of the porous support or introducing these additives to the inside of the porous support also is not particularly limited. For example, when the chelate dye solution is applied to the porous support by an impregnation method, a coating method, a printing method, a spraying method, an ink-jet method, or the like, it is only necessary to cause the additive to be present in the chelate solution. The concentration of the additive in the solution is not particularly limited, and can be set as appropriate. It is to be noted that a mechanism of the bond between these additives and the porous support is not always clear and also is not particularly limited.

A configuration of the dry testing tool of the present invention is not particularly limited, and is, for example, preferably, that when a surface of the porous support to which a chelate dye is bound is an upper surface, the water absorbing layer further is provided on the lower surface of the porous support, and the porous support and the water absorbing layer are at least partially in contact with each other. In the case as described above, the water absorbing layer is not particularly limited, and can be, for example, any of a filter paper, a glass fiber filter paper, a nonwoven fabric, a sponge, and a lattice structure showing a capillary action, or the combination thereof. At that time, more preferably, the porous support and the water absorbing layer are fixed with the holder, and the holder is provided with a through bore for allowing the sample to pass through from the upper side thereof, and for measuring a developed color intensity of a composite of a chelate dye and a metal formed on the surface of the porous support.

Another preferred configuration of the dry testing tool of the present invention may be that the surface of the porous support to which a chelate dye is bound is a lower surface, the water absorbing layer is further provided on the lower surface of the porous support, the water absorbing layer has a ring shape hollowed the middle thereof out, and the porous support is in contact with the water absorbing layer as covering the hollowed portion of the ring shape. At that time, preferably, the porous support and a water absorbing layer are fixed with the holder, the holder allows a liquid sample to pass through from the upper side thereof, and a developed color intensity of the porous support is measured from a lower side thereof through the hollowed portion of the water absorbing layer.

Yet another preferred example of the application of the dry testing tool of the present invention may be that, without using the above described water absorbing layer, only the porous support is fixed on the tip of a reed-shaped base substrate. In this dry testing tool, a metal in a liquid sample can be measured by immersing at least a part of the porous support to which a chelate dye is fixed, then taking it out after the elapse of a certain period of time, and measuring a developed color intensity of a composite of a chelate dye and a metal formed on the surface of the porous support.

When the thickness of the porous support is sufficiently large, the porous support also can serve as the water absorbing layer without being provided with the water absorbing layer, for example. A material of such porous support having a large thickness is not particularly limited and can be, for example, each material described above. The material is preferably a sponge and particularly preferably a sponge of PVA. In the case of such porous support, preferred examples of the method for causing the chelate dye to be bound to the porous support by a hydrophobic bond include methods for causing the chelate dye to bind only to the surface of the porous support by spraying, coating, and the like. The reason is that the large amount of chelate dye is needed in the case of impregnation method because the chelate dye is impregnated into a porous support having a large thickness. However, an impregnation method is preferred in the case where the length of a cell is desired to be long to make full use of the thickness of the porous support.

A method for measuring a metal in a liquid sample with dry testing tools of the various aspects that were described above is not particularly limited. The measuring method can be, for example, a method in which, first, a composite of a chelate dye on the surface of the porous support and a metal is caused to be formed by applying a liquid sample to the dry testing tool, and thereafter, the developed color intensity of the composite can be compared with the standard colorimetric table by visual checking, or the reflectance is measured with a spectral reflectometer and can be converted to the concentration from a calibration curve.

The liquid sample preferably is pretreated prior to the application of the liquid sample to the dry testing tool to conduct a measurement with higher sensitivity and specificity. For example, as described above, there is a case where the ease of binding the objective metal ion to the chelate dye varies depending on the pH. Therefore, the pH preferably is adjusted to be a pH with which the objective metal ion is bound easily to the chelate dye in the pretreatment of adding a pH adjuster to the liquid sample. The pH adjuster is not particularly limited as long as it can adjust pH, and various types of inorganic acids, organic acids, inorganic bases, and organic bases can be used. Specific examples thereof include nitric acid, sulfric acid, hydrochloric acid, acetic acid, citric acid, maleic acid, sodium hydroxide, potassium hydroxide, sodium carbonate, sodium bicarbonate, potassium carbonate, potassium hydrogen carbonate, trimethylamine, triethylamine, Good's buffers, and the like. A pretreatment agent used for the pretreatement is not limited to the pH adjuster, and may be any substance. For example, when a reducing substance in a liquid sample (for example, ascorbic acid in urine) has an adverse effect on the measurement, an oxidizing agent preferably is added to the liquid sample as the pretreatment agent. The oxidizing agent is not particularly limited, and examples thereof include potassium iodate, potassium permanganate, hydrogen peroxide, and the like. As the pretreatment agent, a chelate agent that is different kind of chelate agent from the chelate dye bound to the porous support by a hydrophobic bond may be added as a masking agent. By the masking agent, ions of metals except for the objective metal in the liquid sample can be captured (masked), and the sensitivity and the specificity of the dry testing tool to the objective metal ion can be further increased. The chelate agent as the masking agent is not particularly limited, and examples thereof include maleic acid, citric acid, EDTA, CyDTA, IDA, EDDP, HIDA, DHEG, GEDTA, and the like. The masking agent may be used alone or in a combination of two or more of them, and can be selected as appropriate depending on an objective metal to be measured and an objective metal to be masked. For example, since EDTA, CYDTA, and the like easily capture iron and copper, they are suitable for the case where the objective metal is other than iron and copper.

Next, the hydrophobic bond in the dry testing tool of the present invention will be described further specifically. In the present invention, "hydrophobic bond" means the state where the respective hydrophobic regions in the respective molecules are gathered together, and are stabilized. The porous support in the dry testing tool of the present invention is not particularly limited as described above, and is, for example, composed of a polymer compound. The structure of the polymer compound is not particularly limited, and a hydrophilic region and a hydrophobic region are, for example, present in the molecular structure. Further, to artificially increase the hydrophobic region, a substance having hydrophobicity can be added artificially. On the other hand, a substance having a hydrophobic structure and a hydrophilic structure in a molecule is used as the chelate dye. The dry testing tool of the present invention is a dry testing tool in which a strong hydrophobic bond is caused to be formed by causing hydrophobic regions of the porous support and the chelate dye to be molecularly oriented. This hydrophobic bond can be, for example, as described above, caused to be formed very easily.

Metal ligands of the various types of chelate dyes are generally composed of hydrophilic groups such as an OH group, a COOH group, a NH group, a SH group, and the like or combinations thereof. By the relationship between the properties such as electron densities of these ligands, the distance between each ligand, and the like and the properties such as charges, electron orbital states, dimensions of metal nuclei, and the like of the various metals, the specifity is exerted between each metal and each chelate dye. Since the metal ligands in theses chelate dyes have high electron densities and hydrophilicity, these are oriented from the hydrophobic region on the surface of the porous support toward the outside thereof. Preferably, a ligand in a molecule of the chelate dye bound to the porous support can form a metal-chelate dye composite by freely binding to a metal, and color can be developed even when a structure of a dye molecule is changed by a hydrophobic bond.

The principle of the measurement of a metal by the present invention can be, for example, explained as below. That is, the principle is that a metal (for example, a heavy metal) in a liquid sample is sequentially trapped to the metal ligand of the surface of the chelate dye by flowing it on the metal ligand of the surface of the chelate dye bound to the porous support. That is, the metal in the liquid sample is bound to the ligand of the chelate dye bound to the porous support by a hydrophobic bond, and a chelate dye-metal composite is formed. Accordingly, the formed chelate dye-metal composite is being concentrated on the surface of the porous support. Further, at the same time, the specificity of the chelate dye is maintained. In this way, it can be achieved that an electron conjugated system of a metal-chelate dye composite is formed, and a light at the appropriate wavelength (it is not particularly limited and is, for example, 490 nm that is a wavelength at the time of reaction of dithizone and mercury, 550 nm, or the like) is absorbed. That is, specific color development occurs even under the condition in which the chelate dye is fixed on the porous support, and a metal can be measured by observing the developed color.

In this way, a metal-chelate dye composite is formed effectively on the porous support with the chelate dye corresponding to a target metal that is bound to the porous support. Accordingly, the metal is concentrated, and the color is developed strongly in the state where the formed metal-chelated dye composite is bound to the surface of the porous support.

According to the present invention, color development of most of the metal ions occurs on the surface part of the porous support. For example, by the above-described principle, many noise contents other than an objective metal flow out downward (except for an objective surface). Thus, the measurement that is hardly affected by noise contents that are present in an objective sample (for example, urine) can be achieved. According to the dry testing tool of the present invention, measuring a heavy metal in urine that is a biological sample having a very low concentration up to about 1 µg/kg (or 1 µg/L) with high sensitivity also can be achieved.

According to the dry testing tool or the measuring method of the present invention, it can be expected to measure heavy metals easily in drinking water that is the first cause of consumption in human, soil or plants grown in the soil, and animals consumed the plants and to previously avoid consuming the contaminated food or producing agricultural crops in the contaminated soil. If heavy metals can be measured easily, it also becomes possible to return the contaminated soil and the waste in the deep earth. More actively, the measurement of heavy metals according to the present invention can be an indicator of studies of the technology for recovering these heavy metals.

The developed color intensity of the composite of a chelate dye and a metal on the surface of the porous support may be measured by a method in which the developed color intensity is measured by comparing it with the standard colorimetric table by visual contact or also a method in which a reflectance is measured with a spectral reflectometer, and the measured reflectance is converted to the concentration from the standard curve.

In the dry testing tool or measuring method of the present invention, a sample that is to be an objective sample is not particularly limited. For example, a clinical liquid sample selected from the group consisting of urine, blood, breast milk, hair, spinal fluid, saliva, and decomposed matters thereof can be used as the sample.

Further, for example, an environmental liquid sample selected from the group consisting of extracted liquid from food, extracted liquid from soil, a raw material, a coating material, extracted liquid from a container, waste liquid, sewage water, and seawater can be used as the sample.

An embodiment of the dry testing tool in the present invention is not particularly limited, and can be used for any application as described above. However, one embodiment is for easily measuring heavy metals that are present in urine that is collected particularly easily as biological sample. Since very trace amounts of heavy metals (10 µg/kg or less or 10 µ/L or less) are present in urine, they cannot be measured using only a metal-chelate dye as usual. In order to measure such trace amounts of metals with high sensitivity, in theory, sensitivity cannot be increased unless a molecular extinction coefficient of the metal chelate dye is increased by adding a few more digits. The molecular extinction coefficients of presently commercially available chelate dyes are 10⁵ level. However, when the trace amount of a metal in a sample is measured using a metal chelate dye with this sensitivity, some kind of concentration of a liquid sample is necessary. In the case of urine as a liquid sample, when the urine is concentrated by evaporating the fluid in the urine by heating or reducing the pressure, not only the target metal, but also other metals, salts, proteins, and organic substances are concentrated. That is, a noise content relating to a S/N ratio also is extremely concentrated, and a signal is affected by the noise content.

According to the present invention, only a target component can be very specifically concentrated by, for example, the concentration mechanism described above without concentrating the noise content. That is, in the present invention, in order to concentrate only the target component, the chelete dye is caused to be bound strongly to the surface of the porous support by a hydrophobic bond between a hydrophobic region of the porous support in the dry testing tool and a hydrophobic structure in a molecule of the chelate dye. Further, at the same time, a molecular form in which "ligand that is specific to metals" that is present at other regions in the chelate dye molecule can freely coordinate with metals (for example, heavy metals) is being built. According to the present invention, the sensitivity that is 1000 times higher than that in the case of using a metal chelate dye as usual can also be achieved.

### Examples

Hereinafter, examples of the present invention will be explained. In the dry testing tool and the measuring method in the following examples, a measurement of mercury (Hg) that has higher toxicity among metals was conducted, and further, measurements of copper (Cu), cadmium (Cd), zinc (Zn), and aluminum (Al) also were conducted. Note here that the present invention is not limited by the following examples. For example, the present invention can be applied to the various kinds of metals besides the above-described metals by selecting the type and the like of a chelate dye in an appropriate manner. This is obvious for those skilled in the art from the description of the specification of the present invention.

### [Example 1]

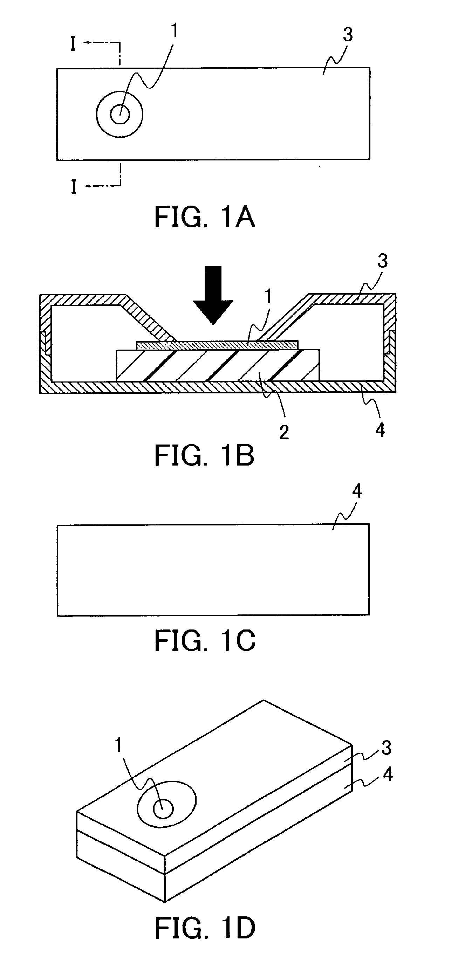

A porous support formed of a dye membrane layer, and further, a dry testing tool having the structure shown in FIGs. 1A to 1D were produced as below. The dry testing tool having the structure shown in FIGs. 1A to 1D was produced. FIG. 1A is a plane view of this dry testing tool, FIG. 1B is a sectional view taken along the line I-I' of FIG. 1A, FIG. 1C is a bottom view, and FIG. 1D is a perspective view.

### (Production of dye membrane layer)

A cellulose acetate membrane having a pore size of 0.2 µm was immersed in a 0.3 mM dithizone-isopropanol solution, and thereafter, the immersed cellulose acetate membrane was pulled out and air-dried at room temperature. Thus, a dye membrane layer was produced. This dye membrane layer was used as a porous support of the dry testing tool.

### (Production of dry testing tool)

As shown in the sectional view in FIG. 1B, a laminated body was formed by causing a porous support 1 formed of the dye membrane layer to be laminated on an upper surface of a water absorbing layer 2 formed of a filter paper. Further, the laminated body was fixed by being sandwiched between an upper holder 3 and a lower holder 4. Thus, the dry testing tool of the present invention was obtained. As shown in FIGs. 1A to 1D, the lower holder 4 has a flat-plate shape at the bottom part, and the laminated body formed of the porous support 1 and the water absorbing layer 2 is laminated thereon. The upper holder 3 has a concave portion, the central part thereof being depressed, and the concave portion may function as a sample holding portion for holding a sample as well as pressing the porous support 1 by the concave portion. Since a part of the upper surface of the porous support 1 is exposed from the central part of the concave portion (the sample holding portion), the part can be in contact with a sample. At the time of measurement, as shown in the arrow in FIG. 1B, the sample is dropped to the porous support 1 exposed from the sample holding portion in the direction from above to below. It is to be noted that the arrow indicates the direction of dropping the sample as well as the direction of measuring a reflectance optically.

### (Measuring method)

The urine samples containing 0, 5, 10, 20, 50, and 100 µg/L mercury, respectively, were prepared, and nitric acid then was added thereto so that the pHs thereof become 3 or less. 500 µL of each urine sample was dropped on the concave portion (the sample holding portion) of the upper holder of the dry testing tool described in Example 1. The dry testing tool was allowed to stand still for 15 to 20 minutes, and the urine sample was almost completely drawn into the water absorbing layer 2 with the capillary function. Then, color from orange to pink was developed on the upper surface of the dye membrane layer (the porous support) 1 depending on the concentration of mercury. The spot with the developed color was subjected to observation by a visual check and a measurement with a spectral reflectometer. The measurement with the spectral reflectometer was conducted by measuring a reflectance of a light at the wavelength of 490 nm. The results will be shown in Table 1 below.

**[Table 1]**

| Mercury concentration (µg/L) | Visual color tone | Reflectance |
|---|---|---|
| 0 | blue | 85% |
| 5 | blue to light orange | 81% |
| 10 | light blue to orange | 75% |
| 20 | orange | 69% |
| 50 | slightly deep orange | 61% |
| 100 | deep orange to pink | 50% |

As shown in Table 1 above, according to Example 1 of the present invention, the observation by a visual check and the measurement of a reflectance with an optical device could be conducted easily in a short time with urine having a mercury concentration in the range of from 0 to 100 µg/L.

### [Example 2]

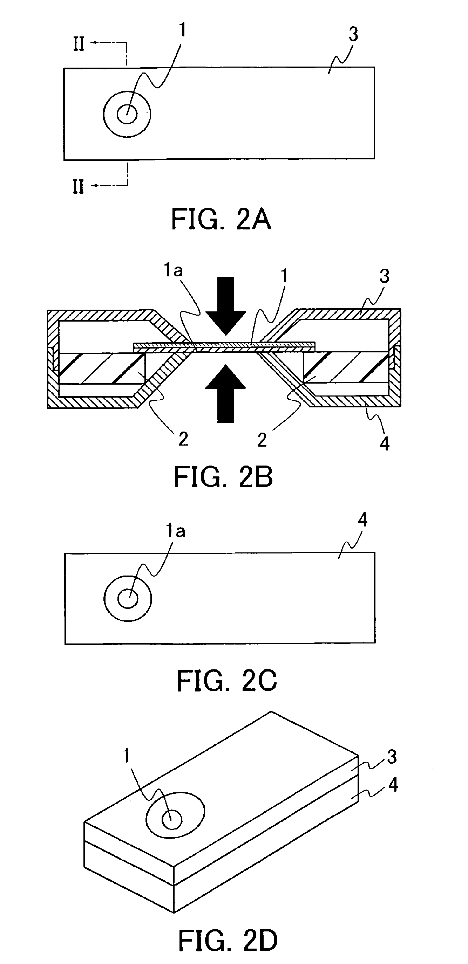

A porous support formed of a dye membrane layer and further, a dry testing tool having the structure shown in FIGs. 2A to 2D were produced as below. FIG. 2A is a plane view of this dry testing tool, FIG. 2B is a sectional view taken along the line I-I' of FIG. 2A, FIG. 2C is a bottom view, and FIG. 2D is a perspective view.

### (Production of dye layer)

A 0.3 mM dithizone-isopropanol solution was sprayed evenly onto one side of a cellulose acetate membrane having a pore size of 0.2 µm using a sprayer, and this then was air-dried at room temperature. Thus, layers of dye were formed on the side of the membrane layer, and a membrane layer having the dye layer was produced. This membrane layer having the dye layer was used as a porous support of a dry testing tool.

### (Production of dry testing tool)

As shown in the sectional view in FIG. 2B, a laminated body was formed by causing a membrane layer having the dye layer (porous support) 1 to be laminated on a ring-shaped water absorbing layer 2 formed of a filter paper as covering the bore of the ring and facing the dye layer 1a down. Further, as shown in FIG. 2B, the ring-shaped water absorbing layer 2 was sealed in a ring-shaped upper holder 3 and lower holder 4, and the membrane layer having the dye layer (porous support) 1 was sandwiched between the ring-shaped upper holder 3 and lower holder 4. Thus, the dry testing tool of the present invention was produced. As shown in FIGs. 2A to 2D, the upper holder 3 and the lower holder 4 each have a concave portion, the central part thereof being depressed, and the porous support 1 can be pressed by the concave portion. The concave portion of the upper holder 3 also functions as a sample holding portion for holding a sample. Since a part of the upper surface of the porous support 1 is exposed from the central part of the concave portion (the sample holding portion) of the upper holder 3, the part can be in contact with a sample. Since a part of the lower surface (dye layer 1a) of the porous support 1 is exposed from the central part of the concave portion of the lower holder 4, an optical measurement of a reflectance can be conducted. At the time of measurement, as shown in the down-pointing arrow in FIG. 2B, the sample is dropped to the porous support 1 exposed from the sample holding portion in the direction from above to below. Further, as shown in the up-pointing arrow in FIG. 2B, light irradiation is conducted from below toward the lower surface (dye layer 1a) of the porous support 1 when the optical reflectance is measured.

### (Measuring method)

0, 10, 50, 100, 500, or 1000 mg of soil containing 1000 µg/kg mercury was collected into a 15 ml-capacity commercially available cuvette with a lid. 10 ml of diluted nitric acid was added thereto so that pHs thereof become 3 or less, and they each were then mixed vigorously by shaking the cuvette. They were allowed to stand still for about 10 minutes, and thereafter, 1000 µL of each sample extracted from the supernatant of each cuvette was dropped on the concave portion (sample holding portion) of the upper holder of the dry testing tool. The dry testing tool was allowed to stand still for 5 to 10 minutes, and the extracted sample was almost completely drawn into the water absorbing layer 2. Color from light pink to deep pink was developed on the dye layer 1a that was at the lower surface of the membrane layer having the dye layer (the porous support) 1 depending on the concentration of mercury. The relationship between the mass of the collected soil and the concentration of mercury in the extracted sample is as shown in Table 2 below. The spot with the developed color was subjected to an observation by a visual check and a measurement with a spectral reflectometer from the lower surface. The measurement with the spectral reflectometer was conducted by measuring a reflectance of a light at the wavelength of 490 nm. The results will be shown in Table 3 below.

**[Table 2]**

| Mass of soil | Mercury concentration (µg/kg) |
|---|---|
| 0 mg | 0 |
| 10 mg | 1 |
| 50 mg | 5 |
| 100 mg | 10 |
| 500 mg | 50 |
| 1000 mg | 100 |

**[Table 3]**

| Mercury concentration (µg/kg) | Color tone | Reflectance |
|---|---|---|
| 0 | blue | 91% |
| 1 | blue to light pink | 89% |
| 5 | light blue to pink | 86% |
| 10 | pink | 80% |
| 50 | deep pink | 70% |
| 100 | strongly reddish pink | 55% |

As shown in Table 3, according to Example 2 of the present invention, the observation by a visual check and the measurement of a reflectance with an optical device could be conducted with soil having a mercury concentration in the range of from 0 to 100 µg/kg. Specifically, although suspended matters and stained fine clay are present in soil, the measurement was less affected by the clay and could be conducted with high accuracy according to the dry testing tool shown in FIG. 2 of the present invention. This was because the solid was filtered when the extracted sample was passed through a membrane having a pore size of 0.2 µm that is on the upper surface of the membrane layer having a dye layer (porous support) 1, residua was filtered and separated to the lower surface whose reflectance should be measured, and the influence caused by the solid (foreign substance) was reduced.

### [Example 3]

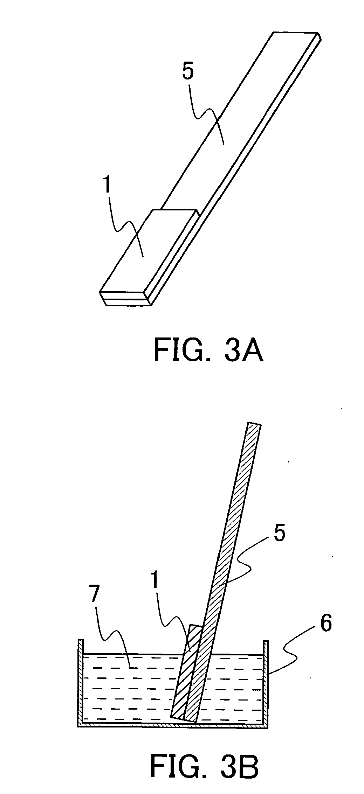

A porous support formed of a dye membrane layer and further, a dry testing tool having the structure shown in FIG. 3A were produced as below.

### (Production of dye membrane layer)

A cellulose acetate membrane having a pore size of 0.2 µm was immersed in a 0.3 mM dithizone-isopropanol solution, and thereafter, the immersed cellulose acetate membrane was pulled out and air-dried at room temperature. Thus, a dye membrane layer was produced. This dye membrane layer was used as a porous support of the dry testing tool.

### (Production of dry testing tool)

A double-faced tape was affixed to one side of the dye membrane layer (porous support) 1. This was affixed in the vicinity of the tip of the 1 mm-thick base material 5 made from polyethylene terephthalate that was cut to be a reed shape as shown in FIG. 3A. Thus, a dry testing tool was completed. It is to be noted that the double-faced tape is not shown in FIG. 3A for simplifying the figure.

### (Measuring method)

0, 10, 50, 100, 500, or 1000 mg of soil containing 1000 µg/kg mercury was collected to a 15-ml capacity commercially available cuvette with a lid. 10 ml of diluted nitric acid was added thereto so that pHs thereof become 3 or less, and they each were then mixed vigorously by shaking the cuvett. As a liquid sample, 1 ml of each turbid solution was collected to a sample cup so that a solution level becomes 3 mm from the bottom surface of the sample cup. Further, as shown in FIG. 3B, the dry testing tool was allowed to stand still in the sample cup 6 in the state with the dye membrane layer (porous support) 1 down. Thus, at least a part of the dye membrane layer (porous support) 1 was immersed in the liquid sample 7. The dry testing tool was allowed to stand still for 30 to 60 minutes as it was and thereafter was pulled out from the sample cup. Color from light pink to deep pink was developed on the 3 mm-thick part immersed in the liquid sample 7 of the dye membrane layer (porous support) 1 depending on the concentration of mercury. The relationship between the mass of the collected soil and the concentration of mercury in the extracted sample is as shown in Table 4 below. The developed color region was subjected to an observation by a visual check and a measurement with a spectral reflectometer. The measurement with the spectral reflectometer was conducted by measuring a reflectance of a light at the wavelength of 490 nm. The results will be shown in Table 5 below.

**[Table 4]**

| Mass of soil | Mercury concentration (µg/kg) |
|---|---|
| 0 mg | 0 |
| 10 mg | 1 |
| 50 mg | 5 |
| 100 mg | 10 |
| 500 mg | 50 |
| 1000 mg | 100 |

**[Table 5]**

| Mercury concentration (µg/kg) | Color tone | Reflectance |
|---|---|---|
| 0 | blue | 95% |
| 1 | blue to light pink | 93% |
| 5 | light blue to pink | 90% |
| 10 | pink | 82% |
| 50 | deep pink | 75% |
| 100 | strongly reddish pink | 63% |

As shown in Table 5, according to Example 3 of the present invention, the observation by a visual check and the measurement of a reflectance with an optical device could be conducted with soil having a mercury concentration in the range of from 0 to 100 µg/kg. In Example 3 of the present invention, the immersing time of the dry testing tool in the liquid sample was 30 to 60 minutes which was a little longer than those in Examples 1 and 2. However, a measurement with high accuracy could be conducted even when the liquid sample was cloudy due to soil and the like. Further, in Example 3 of the present invention, the volumetric capacity of the liquid sample collected to the sample cup from the cuvette was 1 ml. However, according to the dry testing tool having the form such as in Example 3, the amount of a sample is specified depending on the longitudinal volumetric capacity of a membrane. Therefore, there is an advantage that the volumetric capacity of the liquid sample collected from the cuvette to the sample cup is not required to be strictly specified.

### [Example 4]

### (Production of dye membrane layer)

A dye membrane layer was produced in the same manner as in Example 1 except that a cellulose acetate membrane having a pore size of 3.0 µm was used instead of the cellulose acetate membrane having a pore size of 0.2 µm.

### (Production of dry testing tool)

A dry testing tool having the structure shown in FIGs. 1A to 1D was produced in the same manner as in Example 1 except that the dye membrane layer described in Example 4 was used as a porous support.

### (Measuring method)

The urine samples containing 0, 10, 50, and 100 µg/L mercury, respectively, were prepared, maleic acid then was added thereto so that pHs thereof become 3 or less, and further, 0.5 mmol/L potassium iodate was added thereto. 200 µl of each urine sample was dropped on the concave portion (the sample holding portion) of the upper holder of the dry testing tool that was described in Example 4. The dry testing tool was allowed to stand still for 1 to 5 minutes, and the urine sample was almost completely drawn into the water absorbing layer 2 with the capillary function. Then, color from light blue to deep orange was developed on the upper surface of the dye membrane layer (the porous support) 1 depending on the concentration of mercury. The spot with the developed color was subjected to an observation by a visual check and a measurement with a spectral reflectometer. The measurement with the spectral reflectometer was conducted by measuring a reflectance of a light at the wavelength of 490 nm. The results will be shown in Table 6 below.

**[Table 6]**

| Mercury concentration (µg/L) | Color tone | Reflectance |
|---|---|---|
| 0 | blue | 75% |
| 10 | light blue to orange | 70% |
| 50 | slightly deep orange | 62% |
| 100 | deep orange | 50% |

As shown in Table 6 above, according to Example 4 of the present invention, the observation by a visual check and the measurement of a reflectance with an optical device could be conducted easily in a short time with urine having a mercury concentration in the range of from 0 to 100 µg/L. The measurement sensitivity was almost the same as in Example 1. Further, since the-pore size of the porous support was 3 µm that was longer than that in Example 1, the time required for being drawn into the water absorbing layer was 1 to 5 minutes which was shorter than that in Example 1. Thus, a more rapid measurement could be conducted.

### [Example 5]

### (Production of dye membrane layer)

A nonwoven fabric of polyester having a pore size of 2.0 µm (produced by Advantec Co., Ltd., product named "CMF') was immersed in a 0.3 mM dithizone-isopropanol solution and was thereafter pulled out and air-dried at room temperature. Thus, a dye membrane layer was produced. This dye membrane layer was used as a porous support of the dry testing tool. It is to be noted that the CMF (product name) produced by Advantec Co., Ltd. is a porous filter (coated type filter), which is the nonwoven fabric of polyester coated with cellulose acetate.

### (Production of dry testing tool)

A dry testing tool having the structure shown in FIGs. 1A to 1D was produced in the same manner as in Example 1 except that the dye membrane layer described in Example 5 was used as a porous support.

### (Measuring method)

Urine samples were prepared in the same manner as in Example 4. Further, an observation by a visual check and a measurement with a spectral reflectometer at 490 nm were conducted by the same measuring method as in Example 4 except that the dry testing tool described in Example 5 was used as a dry testing tool. The time required for being drawn into the water absorbing layer 2 was 1 to 5 minutes. Color from light blue to deep orange was developed on the upper surface of the dye membrane layer (the porous support) 1 depending on the concentration of mercury. The results will be shown in Table 7 below.

**[Table 7]**

| Mercury concentration (µg/L) | Color tone | Reflectance |
|---|---|---|
| 0 | blue | 75% |
| 10 | light blue to light orange | 69% |
| 50 | orange | 62% |
| 100 | slightly deep orange | 54% |

As shown in Table 7 above, according to Example 5 of the present invention, the observation by a visual check and the measurement of a reflectance with an optical device could be conducted easily in a short time with urine having a mercury concentration in the range of from 0 to 100 µg/L. The measurement sensitivity was almost the same as in Example 4, and the time required for being drawn into a water absorbing layer was also the same as in Example 4. Thus, a rapid measurement could be conducted.

### [Example 6]

### (Production of dye membrane layer)

A dye membrane layer was produced in the same manner as in Example 5 except that a nonwoven fabric of polyester having a pore size of 10.0 µm was used instead of the nonwoven fabric of polyester having a pore size of 2.0 µm. This dye membrane layer was used as a porous support of a dry testing tool.

### (Production of dry testing tool)

A dry testing tool having the structure shown in FIGs. 1A to 1D was produced in the same manner as in Example 1 except that the dye membrane layer described in Example 6 was used as a porous support.

### (Measuring method)

Urine samples were prepared in the same manner as in Example 4. Further, an observation by a visual check and a measurement with a spectral reflectometer at 490 nm were conducted by the same measuring method as in Example 4 except that the dry testing tool described in Example 6 was used as a dry testing tool. The time required for being drawn into the water absorbing layer 2 was 1 to 5 minutes. Color from light blue to deep orange was developed on the upper surface of the dye membrane layer (the porous support) 1 depending on the concentration of mercury. The results will be shown in Table 8 below.

**[Table 8]**

| Mercury concentration (µg/L) | Color tone | Reflectance |
|---|---|---|
| 0 | blue | 75% |
| 10 | light blue to light orange | 70% |
| 50 | orange | 64% |
| 100 | slightly deep orange | 55% |

As shown in Table 8 above, according to Example 6 of the present invention, the observation by a visual check and the measurement of a reflectance with an optical device could be conducted easily in a short time with urine having a mercury concentration in the range of from 0 to 100 µg/L. The measurement sensitivity was almost the same as in Examples 4 and 5, and the time required for being drawn into a water absorbing layer was the same as in Examples 4 and 5. Thus, a rapid measurement could be conducted.

### [Example 7]

Copper (Cu) ions were measured instead of mercury (Hg) ions using the same dry testing tool as in Example 1. A producing method for a dye membrane (a porous support) was the same as in Example 1 except that a 0.3 mM dithizone-isopropanol solution was sprayed evenly onto one side of a cellulose acetate membrane having a pore size of 0.2 µm using a sprayer instead of immersing the cellulose acetate membrane having a pore size of 0.2 µm in the 0.3 mM dithizone-isopropanol solution. A producing method for a dry testing tool was the same as in Example 1 except that the dye membrane (the porous support) described in Example 7 was placed in such a manner that the surface sprayed with the dye is faced up.

### (Measuring method)

The urine samples containing 0, 100, 500, and 1000 µg/L copper, respectively, were prepared, and nitric acid was then added thereto so that pHs thereof become 3 or less. 200 µl of each urine sample was dropped on the concave portion (the sample holding portion) of the upper holder of the dry testing tool described in Example 7. The dry testing tool was allowed to stand still for 15 to 20 minutes, and the urine sample was almost completely drawn into the water absorbing layer 2 with the capillary function. Then, color from light blue to pink was developed on the upper surface of the dye membrane layer (the porous support) 1 depending on the concentration of mercury. The spot with the developed color was subjected to an observation by a visual check and a measurement with a spectral reflectometer. The measurement with the spectral reflectometer was conducted by measuring a reflectance of a light at the wavelength of 550 nm. The results will be shown in Table 9 below.

**[Table 9]**

| Copper concentration (µg/L) | Color tone | Reflectance |
|---|---|---|
| 0 | blue | 71% |
| 100 | light blue to light pink | 66% |
| 500 | light pink | 55% |
| 1000 | pink | 46% |

As shown in Table 9 above, according to Example 7 of the present invention, the observation by a visual check and the measurement of a reflectance with an optical device could be conducted easily in a short time with urine having a copper ion concentration in the range of from 0 to 1000 µg/L.

### [Example 8]

### (Production of dye membrane layer)

A dye membrane layer was produced in the same manner as in Example 4 using a cellulose acetate membrane having a pore size of 3.0 µm.

### (Production of dry testing tool)

A dry testing tool having the structure shown in FIGs. 1A to 1D was produced in the same manner as in Example 4 using the dye membrane layer described in Example 8 as a porous support.

### (Measuring method)

The urine samples containing 0, 100, 500, and 1000 µg/L copper, respectively, were prepared, maleic acid then was added thereto so that pHs thereof become 3 or less, and further, 0.5 mmol/L potassium iodate was added thereto. 200 µL of each urine sample was dropped on the concave portion (the sample holding portion) of the upper holder of the dry testing tool described in Example 8. The dry testing tool was allowed to stand still for 1 to 5 minutes, and the urine sample was almost completely drawn into the water absorbing layer 2 with the capillary function. Then, color from light blue to pink was developed on the upper surface of the dye membrane layer (the porous support) 1 depending on the concentration of mercury. The spot with the developed color was subjected to an observation by a visual check and a measurement with a spectral reflectometer. The measurement with the spectral reflectometer was conducted by measuring a reflectance of a light at the wavelength of 550 nm. The results will be shown in Table 10 below.

**[Table 10]**

| Copper concentration (µg/L) | Color tone | Reflectance |
|---|---|---|
| 0 | blue | 70% |
| 100 | light blue to light pink | 64% |
| 500 | light pink | 56% |
| 1000 | pink | 47% |

As shown in Table 10 above, according to Example 8 of the present invention, the observation by a visual check and the measurement of a reflectance with an optical device could be conducted easily in a short time with urine having a copper concentration in the range of from 0 to 1000 µg/L. The measurement sensitivity was almost the same as in Example 7. Further, since the pore size of the porous support was 3 µm, which was larger than that in Example 7, the time required for being drawn into the water absorbing layer was 1 to 5 minutes, which was shorter than that in Example 7. Thus, a more rapid measurement could be conducted.

### [Example 9]

Cadmium (Cd) ions were measured instead of mercury (Hg) ions using the same dry testing tool as in Example 1. A producing method for a dye membrane and a producing method for a dry testing tool were the same as in Example 1 except that a 0.3 mM dithizone-isopropanol solution was evenly sprayed onto one side of a cellulose acetate membrane having a pore size of 0.2 µm using a sprayer instead of immersing the cellulose acetate membrane having a pore size of 0.2 µm in the 0.3 mM dithizone-isopropanol solution. A producing method of a dry testing tool was the same as in Example 1 except that the dye membrane (the porous support) was placed in such a manner that the surface sprayed with the dye is faced up.

### (Measuring method)

The urine samples containing 0, 100, 500, and 1000 µg/L cadmium, respectively, were prepared, and a pH adjuster then was added thereto so that pHs thereof become about 7. 200 µL of each urine sample was dropped on the concave portion (the sample holding portion) of the upper holder of the dry testing tool described in Example 9. The dry testing tool was allowed to stand still for 15 to 20 minutes, and the urine sample was almost completely drawn into the water absorbing layer 2 with the capillary function. Then, color from light blue to pink was developed on the upper surface of the dye membrane layer (the porous support) 1 depending on the concentration of mercury. The spot with the developed color was subjected to an observation by a visual check and a measurement with a spectral reflectometer. The measurement with the spectral reflectometer was conducted by measuring a reflectance of a light at the wavelength of 550 nm. The results will be shown in Table 11 below.

**[Table 11]**

| Cd concentration (µg/L) | Color tone | Reflectance |
|---|---|---|
| 0 | blue | 73% |
| 100 | light blue to light pink | 68% |
| 500 | light pink | 61% |
| 1000 | pink | 56% |

As shown in Table 11 above, according to Example 9 of the present invention, the observation by a visual check and the measurement of a reflectance with an optical device could be conducted easily in a short time with urine having a cadmium ion concentration in the range of from 0 to 1000 µg/L.

### [Example 10]

Zinc (Zn) ions were measured instead of mercury (Hg) ions using the same dry testing tool as in Example 1. A producing method for a dye membrane (a porous support) was the same as in Example 1 except that a 0.3 mM dithizone-isopropanol solution was evenly sprayed onto one side of a cellulose acetate membrane having a pore size of 0.2 µm using a sprayer instead of immersing the cellulose acetate membrane having a pore size of 0.2 µm in the 0.3 mM dithizone-isopropanol solution. A producing method for a dry testing tool was the same as in Example 1 except that the dye membrane (the porous support) was placed in such a manner that the surface sprayed with the dye is faced up.

### (Measuring method)

The urine samples containing 0, 100, 500, and 1000 µg/L zinc, respectively, were prepared, and a pH adjuster then was added thereto so that pHs thereof become about 7. 200 µL of each urine sample was dropped on the concave portion (the sample holding portion) of the upper holder of the dry testing tool described in Example 10. The dry testing tool was allowed to stand still for 15 to 20 minutes, and the urine sample was almost completely drawn into the water absorbing layer 2 with the capillary function. Then, color from light blue to pink was developed on the upper surface of the dye membrane layer (the porous support) 1 depending on the concentration of zinc. The spot with the developed color was subjected to an observation by a visual check and a measurement with a spectral reflectometer. The measurement with the spectral reflectometer was conducted by measuring a reflectance of a light at the wavelength of 550 nm. The results will be shown in Table 12 below.

**[Table 12]**

| Zinc concentration (µg/L) | Color tone | Reflectance |
|---|---|---|
| 0 | blue | 75% |
| 100 | light blue to light pink | 68% |
| 500 | light pink | 59% |
| 1000 | pink | 52% |

As shown in Table 12 above, according to Example 10 of the present invention, the observation by a visual check and the measurement of a reflectance with an optical device could be conducted easily in a short time with urine having a zinc ion concentration in the range of from 0 to 1000 µ/L.

### [Example 11]

### (Production of dye membrane layer)

An isopropanol solution containing 1 mM 2-(5-bromo-2-pyridylazo)-5-diethylaminophenol (5-Br-PADAP) was evenly sprayed onto one side of mixed cellulose having a pore size of 0.1 µm (mixture of cellulose acetate and cellulose nitrate, Mixed Cellulose Esters (MCE) Membrane Filters (product name) produced by Advantech Co., Ltd.) using a sprayer, and the mixed cellulose then was air-dried at room temperature. Thus, a dye membrane layer was produced. This dye membrane layer was used as a porous support of a dry testing tool.

### (Production of dry testing tool)

A dry testing tool having the structure shown in FIG. 1 was produced in the same manner as in Example 1 except that the dye membrane layer described in Example 11 was used as a porous support, and was placed in such a manner that the surface sprayed with the dye is faced up.

### (Measuring method)

Urine samples containing zinc were prepared in the same manner as in Example 10 except that pHs thereof were adjusted so as to be 10 by adding lithium hydroxide (LiOH). Further, an observation by a visual check and a measurement with a spectral reflectometer at 550 nm were conducted by the same measuring method as in Example 10 except that the dry testing tool described in Example 11 was used as a dry testing tool and the amount of each urine sample to be dropped was 15 µL. The time required for being drawn into the water absorbing layer 2 was 15 to 20 minutes. Color from light blue to pink was developed on the upper surface of the dye membrane layer (the porous support) 1 depending on the concentration of mercury. The measurement results will be shown in Table 13 below.

**[Table 13]**

| Zinc concentration (µg/L) | Color tone | Reflectance |
|---|---|---|
| 0 | blue | 75% |
| 100 | light blue to light pink | 68% |
| 500 | light pink | 59% |
| 1000 | pink | 52% |

As shown in Table 13 above, according to Example 11 of the present invention, the observation by a visual check and the measurement of a reflectance with an optical device could be conducted easily in a short time with urine having a zinc ion concentration in the range of from 0 to 1000 µg/L.

### [Example 12]

Zinc ions were measured using samples prepared from water instead of urine samples and the same dry testing tool as in Example 11.

### (Measuring method)

Aqueous solutions containing 0, 10, 50, and 100 µg/L zinc ions, respectively, were prepared by solving ZnCl₂ in distilled water, and they were used as samples. Zinc ions were measured in the same manner as in Example 11 using 15 µl of each sample. The result showed that an observation by a visual check and a measurement of a reflectance with an optical device could be conducted easily in a short time with water having a zinc ion concentration in the range of from 0 to 1000 µ/L.

### [Example 13]

### (Production of dye membrane layer)

An isopropanol solution containing 1mM stilbazo (4,4'-bis(3,4-dihydroxyphenylazo)-2,2'-stilbenedisulfonic acid diammonium) and 1% by mass cetyltrimethylammonium bromide (CTAB) was evenly sprayed onto one side of mixed cellulose having a pore size of 0.8 µm (mixture of cellulose acetate and cellulose nitrate, Mixed Cellulose Esters (MCE) Membrane Filters (product name) produced by Advantech Co., Ltd.) using a sprayer. The mixed cellulose then was air-dried at room temperature. Thus, a dye membrane layer was produced. This dye membrane layer was used as a porous support of a dry testing tool.

### (Production of dry testing tool)

A dry testing tool having the structure shown in FIGs. 1A to 1D was produced in the same manner as in Example 1 except that the dye membrane layer described in Example 13 was used as a porous support and was placed in such a manner that the surface sprayed with the dye is faced up.

### (Measuring method)

The urine samples containing aluminum (Al) having the various concentrations of from 0 to 20 µg/L, respectively, were prepared, and an acetate buffer was then added so that pHs thereof become 6.0. The measurement was conducted in the same manner as in Example 1 except that 200 µL of each urine sample and the dry testing tool described in Example 13 were used. The result showed that an observation by a visual check and a measurement of a reflectance with an optical device could be conducted easily in a short time with water having an aluminum ion concentration in the range of from 0 to 20 µg/L.

### [Example 14]

### (Production of dye membrane layer)

An isopropanol solution containing 1% by mass cetyltrimethylammonium bromide (CTAB) was evenly sprayed onto one side of a mixed cellulose membrane having a pore size of 0.8 µm (same as in Example 13) using a sprayer. The mixed cellulose membrane then was air-dried at room temperature. Subsequently, an isopropanol solution containing 1mM stilbazo (4,4'-bis(3,4-dihydroxyphenylazo)-2,2'-stilbenzenedisulfonic acid diammonium) was evenly sprayed onto the side of this mixed cellulose membrane on which the CTAB was sprayed. The mixed cellulose membrane then was air-dried at room temperature. Thus, a dye membrane layer was produced. This dye membrane layer was used as a porous support of a dry testing tool.

### (Production of dry testing tool)

A dry testing tool having the structure shown in FIGs. 1A to 1D was produced in the same manner as in Example 13 except that the dye membrane layer described in Example 14 was used as a porous support and was placed in such a manner that the surface sprayed with the dye is faced up.

### (Measuring method)

The urine samples containing aluminum (Al) having the various concentrations of from 0 to 1000 µg/L, respectively, were prepared, and an acetate buffer then was added thereto so that pHs thereof become 6.0. The measurement was conducted in the same manner as in Example 13 using 200 µL of each urine sample. The result showed that an observation by a visual check and a measurement of a reflectance with an optical device could be conducted easily in a short time with water having an aluminum ion concentration in the range of from 0 to 1000 µg/L.

### [Example 15]

### (Production of dye membrane layer)

An isopropanol solution containing 1mM stilbazo (4,4'-bis(3,4-dihydroxyphenylazo)-2,2'-stilbenedisulfonic acid diammonium) and 1% by mass Triton X-100 (product name) was evenly sprayed onto one side of a mixed cellulose membrane having a pore size of 0.8 µm (same as in Example 13) using a sprayer. The mixed cellulose membrane then was air-dried at room temperature. Thus, a dye membrane layer was produced. This dye membrane layer was used as a porous support of a dry testing tool.

### (Production of dry testing tool)

A dry testing tool having the structure shown in FIGs. 1A to 1D was produced in the same manner as in Example 13 except that the dye membrane layer described in Example 15 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14, and an acetate buffer further was added thereto so that pHs thereof become 6.0. The measurement was conducted by using 200 µL of each urine sample and in the same manner as in Example 13 except that the dry testing tool described in Example 15 were used. The result showed that an observation by a visual check and a measurement of a reflectance with an optical device could be conducted easily in a short time with water having an aluminum ion concentration in the range of from 0 to 1000 µg/L.

### [Example 16]

### (Production of dye membrane layer)

A dye membrane layer was produced in the same manner as in Example 14 except that a pretreatement was conducted using octylamine instead of CTAB. This dye membrane layer was used as a porous support of a dry testing tool.

### (Production of dry testing tool)

A dry testing tool having the structure shown in FIG. 1 was produced in the same manner as in Example 14 except that the dye membrane layer described in Example 16 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 16 were used. The result showed that an observation by a visual check and a measurement of a reflectance with an optical device could be conducted easily in a short time with water having an aluminum ion concentration in the range of from 0 to 1000 µg/L.

### [Example 17]

A dye membrane layer was produced in the same manner as in Example 13 except that a cellulose acetate membrane having a pore size of 1.0 µm was used instead of the mixed cellulose membrane. This dye membrane layer was used as a porous support of a dry testing tool.

### (Production of dry testing tool)

A dry testing tool having the structure shown in FIGs. 1A to 1D was produced in the same manner as in Examples 13 and 14 except that the dye membrane layer described in Example 17 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 17 were used. The result showed that an observation by a visual check and a measurement of a reflectance with an optical device could be conducted easily in a short time with water having an aluminum ion concentration in the range of from 0 to 1000 µg/L.

### [Example 18]

A dye membrane layer was produced in the same manner as in Example 13 except that a cellulose nitrate membrane having a pore size of 0.2 µm was used instead of the mixed cellulose membrane. This dye membrane layer was used as a porous support of a dry testing tool.

### (Production of dry testing tool)

A dry testing tool having the structure shown in FIGs. 1A to 1D was produced in the same manner as in Examples 13 and 14 except that the dye membrane layer described in Example 18 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 18 were used. The result showed that an observation by a visual check and a measurement of a reflectance with an optical device could be conducted easily in a short time with water having an aluminum ion concentration in the range of from 0 to 1000 µg/L.

### [Example 19]

A dye membrane layer was produced in the same manner as in Example 13 except that a water-absorbing filter paper produced by Advantech Co., Ltd. (product name "26-WA") was used instead of the mixed cellulose membrane. This dye membrane layer was used as a porous support of a dry testing tool.

### (Production of dry testing tool)

A dry testing tool having the structure shown in FIGs. 1A to 1D was produced in the same manner as in Examples 13 and 14 except that the dye membrane layer described in Example 19 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 19 were used. The result showed that an observation by a visual check and a measurement of a reflectance with an optical device could be conducted easily in a short time with water having an aluminum ion concentration in the range of from 0 to 1000 µg/L.

### [Example 20]

A dye membrane layer was produced in the same manner as in Example 19 except that octadecylamine was used instead of CTAB. This dye membrane layer was used as a porous support of a dry testing tool.

### (Production of dry testing tool)

A dry testing tool having the structure shown in FIGs. 1A to 1D was produced in the same manner as in Example 19 except that the dye membrane layer described in Example 20 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 20 were used. The result showed that an observation by a visual check and a measurement of a reflectance with an optical device could be conducted easily in a short time with water having an aluminum ion concentration in the range of from 0 to 1000 µg/L.

### [Example 21]

A dye membrane layer was produced in the same manner as in Example 19 except that octadecylsilane was used instead of CTAB. This dye membrane layer was used as a porous support of a dry testing tool.

### (Production of dry testing tool)

A dry testing tool having the structure shown in FIGs. 1A to 1D was produced in the same manner as in Example 19 except that the dye membrane layer described in Example 21 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 21 were used. The result showed that an observation by a visual check and a measurement of a reflectance with an optical device could be conducted easily in a short time with water having an aluminum ion concentration in the range of from 0 to 1000 µg/L.

### [Example 22]

A dye membrane layer was produced in the same manner as in Example 19 except that Triton X-100 (product name) was used instead of CTAB. This dye membrane layer was used as a porous support of a dry testing tool.

### (Production of dry testing tool)

A dry testing tool having the structure shown in FIGs. 1A to 1D was produced in the same manner as in Example 19 except that the dye membrane layer described in Example 22 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 22 were used. The result showed that an observation by a visual check and a measurement of a reflectance with an optical device could be conducted easily in a short time with water having an aluminum ion concentration in the range of from 0 to 1000 µg/L.

### [Example 23]

A dye membrane layer was produced in the same manner as in Example 19 except that SDS (sodium dodecyl sulphate) was used instead of CTAB. This dye membrane layer was used as a porous support of a dry testing tool.

### (Production of dry testing tool)

A dry testing tool having the structure shown in FIGs. 1A to 1D was produced in the same manner as in Example 19 except that the dye membrane layer described in Example 23 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 23 were used. The result showed that an observation by a visual check and a measurement of a reflectance with an optical device could be conducted easily in a short time with water having an aluminum ion concentration in the range of from 0 to 1000 µg/L.

### [Example 24]

### (Production of dye membrane layer)

An isopropanol solution containing 1 mM chromazurol S was caused to be passed through a cellulose acetate membrane by suction filtration using a suction filtration device to a cellulose acetate membrane having a pore size of 1.0 µm, and thereafter, the cellulose acetate membrane was air-dried at room temperature. Thus, a dye membrane layer was produced. This dye membrane layer was used as a porous support of a dry testing tool.

### (Production of dry testing tool)

A dry testing tool having the structure shown in FIGs. 1A to 1D was produced in the same manner as in Example 1 except that the dye membrane layer described in Example 24 was used as a porous support and was placed in such a manner that the surface sprayed with the dye is faced up.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 24 were used. The result showed that an observation by a visual check and a measurement of a reflectance with an optical device could be conducted easily in a short time with water having an aluminum ion concentration in the range of from 0 to 1000 µg/L.

### [Example 25]

A dry testing tool was produced in the same manner as in Example 24 except that a cellulose nitrate membrane having a pore size of 0.2 µm was used instead of the cellulose acetate membrane having a pore size of 1.0 µm.

### (Measuring method)

The urine samples containing aluminum (Al) having the various concentrations of from 0 to 1000 µg/L, respectively, were prepared in the same manner as in Example 14. Further, pHs thereof were adjusted in the same manner as in Example 14 so as to be 6.0. The measurement was conducted by using 200 µL of each urine sample and in the same manner as in Example 14 except that the dry testing tool described in Example 25 were used. The result showed that an observation by a visual check and a measurement of a reflectance with an optical device could be conducted easily in a short time with water having an aluminum ion concentration in the range of from 0 to 1000 µg/L.

### Industrial Applicability

As explained above, according to the dry testing tool or the method for measuring a metal in a sample of the present invention, a target metal can be measured easily, rapidly, and high sensitively by the simple colorimetric method. Further, according to the present invention, the measurement can be conducted anywhere by anyone, and a simple-and-inexpensive measurement without requiring a large device can be achieved, for example.

According to the present invention, the state of accumulation of a heavy metal in a biological sample (hair, blood, or urine) can be grasped, for example. Further, the amount of a heavy metal excreted by the detoxification treatment or the amount of a residual heavy metal after the treatment can be grasped easily.

Furthermore, for example, by easily measuring a heavy metal in food (fishery product, dairy products, or the like), the import of the contaminated food can be stopped in advance, and by checking the safety of the food at the time of delivery, the use of, contaminated food for human consumption can be precluded.

The present invention is not limited to these and can be applied to the various types of samples and applications. For example, it is possible that drinking contaminated water is precluded by measuring heavy metals in water source, ground water and water supply, and heavy metals are not caused to be discharged to the environment by measuring heavy metals in water discharged from factories, research institutions, and the like. For example, can be achieved controlled cultivation of dietary plant in contaminated soil by measuring a heavy metal in soil and determining an effect after collecting actively the heavy metal in the contaminated soil. According to the present invention, for example, since the measurement can be conducted anywhere by anyone, and a simple-and-inexpensive measurement without requiring a large device can be achieved, its usefulness is extremely high.

## Claims

1. A dry testing tool for measuring a metal in a sample, comprising a porous support and a chelate dye,
wherein the chelate dye is bound to the porous support by a hydrophobic bond.

2. The dry testing tool according to claim 1, wherein, by supplying the sample to a surface of the porous support, the metal in the sample is bound to the chelate dye by a chelate bond and a composite of the chelate dye and the metal is formed on the surface of the porous support.

3. The dry testing tool according to claim 1, wherein the porous support is at least one selected from the group consisting of filter papers, sheets, membranes, nonwoven fabrics, woven fabrics, fabrics, and sintered bodies.

4. The dry testing tool according to claim 1, wherein a material of the porous support is at least one selected from the group consisting of cellulose, a cellulose derivative, glasses, and a polymer.

5. The dry testing tool according to claim 4, wherein the cellulose derivative is at least one selected from the group consisting of cellulose nitrate, cellulose acetate, and mixed cellulose.

6. The dry testing tool according to claim 4, wherein the polymer is at least one selected from the group consisting of polycarbonates, polypropylenes, polytetrafluoroethylenes, polyethersulfones, polystyrenes, and polyesters.

7. The dry testing tool according to claim 2, further comprising a porous filter, wherein the porous filter is formed on an upper surface of the porous support, a noise content in the sample is captured by the porous filter by supplying the sample on the upper surface of the porous filter, and the sample passing through the porous filter is supplied on the surface of the porous support.

8. The dry testing tool according to claim 1, wherein the chelate dye is a chelate dye that specifically forms a chelate composite with an objective metal.

9. The dry testing tool according to claim 8, wherein when the objective metal is mercury, the chelate dye is at least one selected from the group consisting of dithizone compounds, thiomichler's compounds, thiothenoyltrifluoroacetone, diphenylcarbazone, zincon, derivates of PADAP, and N-benzoyl-N-phenyl-hydroxylamine.

10. The dry testing tool according to claim 8, wherein
when the objective metal is mercury, the chelate dye is at least one selected from the group consisting of dithizone, thiomichler's ketone, thiothenoyltrifluoroacetone, diphenylcarbazone, zincon, 2-(5-bromo-2-pyridylazo)-5-diethylaminophenol (5-Br-PADAP), and N-benzoyl-N-phenyl-hydroxylamine,
when the objective metal is cadmium, the chelate dye is at least one selected from the group consisting of 2-(5-bromo-2-pyridylazo)-5-diethylaminophenol (5-Br-PADAP), 1-(2-pyridylazo)-2-naphthanol (PAN), dithizone, eriochrome black T, and chromazurol S,
when the objective metal is aluminum, the chelate dye is at least one selected from the group consisting of stilbene-4,4'-bis(1-azo)-3,4-dihydroxybenzene-2,2'-disulfonic acid (stilbazo), chromazurol S, N-phenylbenzohydroxamic acid (BPA), aurine tricarboxylic acid ammonium salt (another name: aluminon), and phenylfluorone,
when the objective metal is zinc, the chelate dye is at least one selected from the group consisting of 2-(5-bromo-2-pyridylazo)-5-diethylaminophenol (5-Br-PADAP), 2-(5-bromo-2-pyridylazo)-5-(N-propyl-N-sulfopropylamino)phenol (5-Br-PAPS), dithizone, 2-(5-nitro-2-pyridylazo)-5-(N-propyl-N-sulfopropylamino)phenol (Nitro-PAPS), PAN, 4-(2-pyridylazo)resorcinol (PAR), phenylfluorone, and zincon,
when the objective metal is lead, the chelate dye is at least one selected from the group consisting of 5,10,15,20-tetraphenyl-21H,23H-porphinetetrasulfonic acid, disulfuric acid (TPPS), dithizone, bromopyrogallol red (BPR), PAR, and xylenol orange,
when the objective metal is copper, the chelate dye is at least one selected from the group consisting of PAN, PAR, PR, TMPyP, XO, bathocuproine, and dithizone,
when the objective metal is cobalt, the chelate dye is 2-nitroso-1-naphthol, and
when the objective metal is chromium, the chelate dye is diphenylcarbazide.

11. The dry testing tool according to claim 1, wherein a surface of the porous support comprises at least a hydrophobic region, and the chelate dye is bound thereto by a hydrophobic bond.

12. The dry testing tool according to claim 11, wherein the hydrophobic region comprises a hydrophobic group.

13. The dry testing tool according to claim 12, wherein the hydrophobic group is at least one selected from the group consisting of alkyl groups, nitro groups, acyl groups, cycloalkyl groups, unsaturated hydrocarbon groups, and phenyl groups.

14. The dry testing tool according to claim 11, wherein the hydrophobic region is formed by introducing a hydrophobic group to the porous support.

15. The dry testing tool according to claim 11, wherein the hydrophobic region is formed by coating the porous support with a hydrophobic substance.

16. The dry testing tool according to claim 1, further comprising a water absorbing layer, wherein the porous support and the water absorbing layer are at least partially in contact with each other, and the sample passing through the porous support can be drawn into the water absorbing layer.

17. The dry testing tool according to claim 16, wherein the water absorbing layer is at least one selected from the group consisting of filter papers, glass fiber filter papers, nonwoven fabrics, sponges, and lattice structures exerting a capillary action, or is a combination thereof.

18. The dry testing tool according to claim 16, wherein when a surface of the porous support to which a chelate dye is bound is an upper surface, the water absorbing layer is placed on the lower surface of the porous support, and the porous support and the water absorbing layer are at least partially in contact with each other.

19. The dry testing tool according to claim 16, further comprising a holder provided with a through bore,
wherein the porous support and the water absorbing layer are fixed with the holder,
the through bore of the holder allows the sample to be passed through from the upper side thereof, and
a developed color intensity of a composite of the chelate dye and the metal formed on a surface of the porous support can be measured.

20. The dry testing tool according to claim 16, wherein the water absorbing layer is a ring-shaped water absorbing layer provided with a through bore, and
when a surface of the porous support to which a chelate dye is bound is a lower surface, the water absorbing layer is placed on the lower surface of the porous support, and the porous support is in contact with the water absorbing layer as covering the through bore part of the ring-shaped water absorbing layer.

21. The dry testing tool according to claim 20, further comprising a holder,
wherein the porous support and the water absorbing layer are fixed with the holder,
the holder allows the sample to be passed through from the upper side thereof to the porous support, and
a developed color intensity of a composite of the chelate dye and the metal formed on the surface of the porous support can be measured from a lower side thereof through the through bore part of the water absorbing layer.

22. A method for measuring a metal in a sample, comprising the steps of:
forming a composite of a chelate dye and a metal in a sample by supplying the sample to a porous support to which a chelate dye is bound by a hydrophobic bond; and
detecting the metal in the sample using developed color of the composite.

23. The measuring method according to claim 22, wherein in the detection step, a developed color intensity of the composite is measured.

24. The measuring method according to claim 23, wherein in the detection step, based on the developed color intensity of the composite, a concentration or an amount of the metal in a sample is measured.

25. The measuring method according to claim 24, wherein developed color of the composite is detected by a visual check, and the concentration of the metal is measured by comparing the developed color with a standard colorimetric table.

26. The measuring method according to claim 25, wherein a reflectance of a porous support is measured, and the concentration of the metal is calculated from a calibration curve.

27. The measuring method according to claim 22, wherein the dry testing tool according to claim 1 is used, and the sample is supplied to the porous support in the dry testing tool.

28. The measuring method according to claim 22, wherein in the forming step, the porous support is immersed in the sample, and then is taken out after the elapse of a certain period of time.

29. The measuring method according to claim 28, wherein the porous support is fixed on a tip of a reed-shaped supporting substrate, and the porous support fixed on the supporting substrate is immersed in the sample.

30. A method for producing the dry testing tool according to claim 1, comprising the steps of:
causing a chelate dye to be bound to a porous support by a hydrophobic bond by causing a solution in which the chelate dye is dissolved to be in contact with a porous support comprising a hydrophobic region; and
drying the porous support.

31. The producing method according to claim 30, wherein a method for causing the solution to be in contact with the porous support is at least one method selected from the group consisting of impregnation methods, coating methods, printing methods, spraying methods, and ink-jet methods.

32. The producing method according to claim 30, wherein a solvent is an organic solvent or a mixed solvent of the organic solvent and water.
